# EUROPEAN PATENT SPECIFICATION

(11) **EP 1 934 390 B1**
(45) Date of publication and mention of the grant of the patent: **13.04.2011**
(21) Application number: 06816703.0
(22) Date of filing: 11.10.2006
(51) Int. Cl.: D01F 9/00, D01F 6/92, C08L 3/02, C08G 63/02, C08G 63/12, D01F 8/14

(54) **WATER STABLE FIBERS AND ARTICLES COMPRISING STARCH, AND METHODS OF MAKING THE SAME**
WASSERSTABILE FASERN UND ARTIKEL MIT STÄRKE, SOWIE HERSTELLUNGSVERFAHREN
FIBRES STABLES A L'EAU ET ARTICLES COMPRENANT DE L'AMIDON, ET LEURS PROCEDES DE FABRICATION

(30) Priority: 11.10.2005 US 725424 P
(43) Date of publication of application: 25.06.2008
(73) Proprietor: The Procter & Gamble Company, Cincinnati, OH 45202 (US)
(72) Inventor: NODA, Isao, Fairfield, Ohio 45014 (US); SATKOWSKI, Michael, M., Oxford, Ohio 45056 (US); ALLEN, William, M., Jr., Liberty Township, Ohio 45044 (US); KNAPMEYER, James, T., Rossmoyne, Ohio 45236 (US)
(74) Representative: Engisch, Gautier
(86) International application number: PCT/US2006/039709
(87) International publication number: WO 2007/044802

(56) References cited:
- EP-A2- 0 327 505
- WO-A-00/55236
- WO-A-93/07213
- WO-A-96/31561
- WO-A-02/090629

## Description

### FIELD OF THE INVENTION

The present invention relates to fibers made from thermoplastic starch compositions, and articles made therefrom. The fibers and articles are water stable, or may be rendered so. The invention also relates to methods of making the fibers and articles.

### BACKGROUND OF THE INVENTION

There have been many attempts to make starch-containing fibers, particularly on a high speed industrial level. However, starch fibers can be much more difficult to produce than films, blow-molded articles, and injection-molded articles containing starch because the material and processing characteristics for fibers are much more stringent. For example, local strain rates and shear rates can be much greater in fiber production than in other processes. Additionally, a homogeneous composition may be required for fiber spinning. For spinning fine fibers, small defects, slight inconsistencies, or non-homogeneity in the melt are typically not acceptable for current, commercially viable processes.

Fibers of degradable material that may comprise starch and polyesters are, for example, described in WO96/31561, WO02/090629 and WO00/55236.

In recent years, attempts have been made to process starch on standard equipment and using existing technology known in the plastic industry. Fibers comprising starch may be desired over conventional plastics for a variety of reasons. Unpredictable fluctuations in price and availability of petroleum and its derivatives have created serious disruptions to the stable supply of petroleum-based polymers used in making synthetic fibers, for example, those based on polyolefins. Starch also has material properties not typically provided by conventional polyolefin plastics, including higher hydrophilicity (such as for improved absorbency), as well as affinity and compatibility with other materials not normally compatible with polyolefins. Starch may, in some forms, also provide consumer-related benefits, like easy disposability and/or flushability, and/or socially and environmentally relevant properties, like bio-sourcing and biodegradability. Starch may also provide a low-cost alternative to conventional petroleum- based materials, for example polypropylene.

In conventional processes, starch is typically combined with one or more plasticizers or other process aids to render it thermoplastic for processing, for example by melt spinning or other melt extrusion techniques. Unfortunately, thermoplastic starch (TPS) is highly susceptible to moisture. In fact, fibers made of TPS can spontaneously pick up atmospheric moisture and become tacky. When placed in water, TPS fibers from conventional starch blends partially or fully disintegrate within hours. Although methods exist for rendering thermoplastic compositions containing starch more water stable including, but not limited the addition of petroleum based polymers, there remains an unmet need for greater water stability in such compositions and in articles made from such compositions.

### SUMMARY OF THE INVENTION

In some embodiments, the present invention relates to a method of making water stable fibers comprising the steps of: a. forming a mixture of: i. destructured starch, preferably destructured in situ; ii. polyhydric alcohol having alcohol functional groups; and iii. acid having at least one functional group selected from the group consisting of: carboxylic acid; carboxylic anhydride; and combinations thereof; b. extruding said mixture through a spinneret at elevated temperature, preferably above 90°C, to form fibers; and c. inducing an ester condensation reaction between at least a portion of said polyhydric alcohol and said acid, wherein water is a product of said reaction and wherein said reaction is induced before or during the formation of the fiber and further wherein as used herein the term water stable means the fibers remain intact after two weeks in 200ml of tap water at room temperature according to the procedure disclosed in the description.

Optionally, the mixture of (a) further comprises a triglyceride. In that case, the method step c) may further comprise inducing a transesterification reaction between polyhydric alcohol and triglyceride.

In another aspect, the present invention relates to a fiber comprising a thermoplastic starch composition, said composition comprising: a. at least 50wt.%, by weight of the composition, of destructured starch, preferably destructured in situ; and b. ester condensation products formed from a chemical reaction in a reactant mixture, said reactant mixture being compounded with said destructured starch and said reactant mixture comprising: i. polyhydric alcohol having alcohol functional groups, preferably selected from the group consisting of: glycerol; glycol; sugar; sugar alcohol; and combinations thereof; more preferably glycerol; and ii. acid with at least one functional group selected from the group consisting of: carboxylic acid; carboxylic acid anhydride; and combinations thereof; said functional groups being present in said reactant mixture in a molar ratio of said alcohol functional groups to said at least one acid functional group of from 1:1 to 200:1_{;} and further wherein said fiber is water stable, which as used herein means the fiber remains intact after two weeks in 200ml of tap water at room temperature according to the procedure disclosed in the description.

In some embodiments, articles are made from the fibers of the present invention. Non- limiting examples of articles in include nonwovens. Specific embodiments include personal hygiene articles, absorbent articles, and packaging.

In general, the present invention provides starch-based compositions, fibers and articles in other forms with improved water stability, and compositions and processes for making such water stable compositions and articles. Water stability may be achieved without requiring the use of petroleum based polymers including, but not limited to, polyolefins. Water stability can provide a variety of consumer-related benefits. The fibers, compositions and processes may provide a low-cost alternative to conventional petroleum-based materials. These and additional advantages will be more apparent in view of the following detailed description.

### DETAILED DESCRIPTION

All percentages, ratios and proportions used herein are by weight percent of the composition, unless otherwise specified. All average values are calculated "by weight" of the composition or components thereof, unless otherwise expressly indicated. "Average molecular weight," or "molecular weight" for polymers, unless otherwise indicated, refers to weight average molecular weight. Weight average molecular weight, unless otherwise specified, is determined by gel permeation chromatography.

"Copolymer" as used herein is meant to encompass copolymers, terpolymers, and other multiple-monomer polymers.

"Reactant" as used herein refers to a chemical substance that is present at the start of a chemical reaction.

"Mixture" as used herein refers to a mixture of two or more of any of a defined group of components, unless otherwise specified.

"Biodegradable" as used herein refers to the ability of a compound to ultimately be degraded completely into CH₄, CO₂ and water or biomass by microorganisms and/or natural environmental factors.

"Fiber" as used herein includes staple fibers, fibers longer than staple fibers that are not continuous, and continuous fibers, which are sometimes referred to in the art as "substantially continuous filaments" or simply "filaments". The method in which the fiber is prepared will determine if the fiber is a staple fiber or a continuous filament.

"Monocomponent fiber" as used herein, refers to a fiber formed from using one or more extruders from only one polymer. This is not meant to exclude fibers formed from one polymer to which small amounts of additives have been added. Additives may be added to the polymer for the purposes of providing the resulting fiber with coloration, antistatic properties, lubrication, hydrophilicity, and the like.

"Multicomponent fiber" as used herein refers to a fiber formed from two or more different polymers that are extruded from separate extruders and spun together to form one fiber.

| | Melt Pressure (psi) | Zone 1 (°C) | Zone 2 (°C) | Zone 3 (°C) | Pressure (psi) | Head (°C) |
|---|---|---|---|---|---|---|
| Set Extruder 1 (°C) TPS | 1400 | 125 | 160 | 170 | 1500 | 175 |
| Set Extruder 2 (°C) PP | 1400 | 165 | 170 | 175 | 1500 | |

Fibers are collected through an attenuating air jet set at 20 psi. A total mass throughput of 0.75 g/hole-min is maintained. Adjusting the ratio of the melt pump speeds can produce sheath core fibers of different sheath thicknesses. The following sheath /core volume ratio is produced:

| Sheath (PP) (% volume) | Core (TPS) (% volume) |
|---|---|
| 5 | 95 |

Webs of approximately 60 grams /m² are bonded via heated calender with diamond shaped pattern (1 mm in width , at 2mm intervals) at 165°C. The webs are dried in a oven at 115°C for 12 hours. A 5cm x 5cm piece of the web is put into 1000ml of tap water and stirred at 30 rpm for 24 hours. The web is removed from the water dried in air for 24 hours then measured. The length and width dimension changes by more than 15% and the web is not essentially intact with missing pieces. The web is said to not display water stability.

"Bicomponent fibers" are one type of multicomponent fiber, and are formed from two different polymers. Bicomponent fibers may sometimes be referred to as "conjugate fibers" or "multicomponent fibers". Bicomponent fibers may be comprised of polymers that are substantially constantly positioned in distinct zones, both across the cross-section of the bicomponent fibers and along their length. Non-limiting examples of such bicomponent fibers include, but are not limited to: sheath/core arrangements, wherein one polymer is surrounded by another; side-by-side arrangements; segmented pie arrangements; or even "islands-in-the-sea" arrangements. Each of the aforementioned polymer arrangements is known in the art of multicomponent (including bicomponent) fibers.

Bicomponent fibers can be splittable fibers; such fibers are capable of being split lengthwise before or during processing into multiple fibers with each of the multiple fibers having a smaller cross-sectional dimension than that of the original bicomponent fiber. Splittable fibers have been shown to produce softer nonwoven webs due to their reduced cross-sectional dimensions. Representative splittable fibers useful in the present invention include type T-502 and T-512 16 segment PET/nylon 6, 2.5 denier fibers, and type T-522 16 segment PET/PP splittable fibers, all of which are available from Fiber Innovation Technology, Johnson City, TN.

"Biconstituent fibers" as used herein, refers to fibers which have been formed from at least two starting polymers extruded as a blend from the same extruder. Biconstituent fibers may have the various polymer components arranged in relatively constantly positioned distinct zones across the cross-sectional area of the fiber and the various polymers are usually not continuous along the entire length of the fiber. In the alternative, biconstituent fibers may comprise a blend, that may be homogeneous or otherwise, of the at least two starting polymers. For example, a bicomponent fiber may be formed from starting polymers which differ only in molecular weight.

The polymers comprising biconstituent fibers may form fibrils, which may begin and end at random along the length of the fiber. Biconstituent fibers may sometimes be referred to as multiconstituent fibers.

The terms "non-round fibers" and "shaped fibers" as used interchangeably herein, refer to fibers having a cross-section that is not circular, and includes, but is not limited to those fibers that are "shaped fibers" and "capillary channel fibers." Such fibers can be solid or hollow, and they can be tri-lobal, delta-shaped, and are preferably fibers having capillary channels on their outer surfaces. The capillary channels can be of various cross-sectional shapes such as "U-shaped", "H-shaped", "C-shaped" and "V-shaped". One preferred capillary channel fiber is T-401, designated as 4DG fiber available from Fiber Innovation Technologies, Johnson City, TN. T-401 fiber is a polyethylene terephthalate (PET polyester). Further examples of shaped fibers of use in the present invention are found in U.S. Pat. Pub. No. 2005/0176326 A1.

The terms "nonwoven web" or "web" are used interchangeably herein, and refer to a layer of individual fibers or threads that are interlaid, but not in an identifiable manner as in a knitted or woven web. Nonwoven webs may be made via processes known in the art, including those that comprise the following non-limiting examples. Fiber laying processes of use may include, but are not limited to: carding; airlaying; and wetlaying. Processes comprising filament spinning from resin and integrated webforming include, but are not limited to: spunbonding; meltblowing; coforming; and forming spunbond-meltblown-spunbond composites. Fiber bonding processes of use may include, but are not limited to: spunlacing (i.e. hydroentanglement); cold calendering; hot calendering; air thru bonding; chemical bonding; needle punching; and combinations thereof.

"Compostable" as used herein refers to a material that meets the following three requirements: (1) the material is capable of being processed in a composting facility for solid waste; (2) if so processed, the material will end up in the final compost; and (3) if the compost is used in the soil, the material will ultimately biodegrade in the soil.

"Comprising" as used herein means that various components, ingredients or steps can be conjointly employed in practicing the present invention. Accordingly, the term "comprising" encompasses the more restrictive terms "consisting essentially of" and "consisting of". The present compositions can comprise, consist essentially of, or consist of any of the required and optional elements disclosed herein.

Markush language as used herein encompasses combinations of the individual Markush group members, unless otherwise indicated.

All percentages, ratios and proportions used herein are by weight percent of the composition, unless otherwise specified. All average values are calculated "by weight" of the composition or components thereof, unless otherwise expressly indicated.

All numerical ranges disclosed herein, are meant to encompass each individual number within the range and to encompass any combination of the disclosed upper and lower limits of the ranges.

The present invention is directed to water stable fibers, articles comprising water stable fibers, and processes for making the same. Within the context of the present specification, "water stable" describes a material that remains intact after two weeks in 200 ml of tap water at room temperature according to the following procedure. 200 ml of tap water are charged to a clean glass container, to which about 0.5 grams of material is added. The material should be in a form that displays an aspect ratio of greater than about 1:20 with a minimum axis no larger than 1 mm. This condition is easily met for fibers of diameter less than 1 mm. Suitably, at least 10 test pieces should be added to the container with water. The container is closed and agitated by an orbital mechanical shaker (for example a Madell Technology ZD-9556, Omaha Nebraska) at 100 rpm for 15 minutes to coat the material with water. After 1 hour, 24 hours, 48 hours, 72 hours and two weeks, the contents are agitated by an orbital mechanical shaker at 100 rpm for 15 minutes. If, after two weeks, the material is still intact, with no disintegration, the material is considered to be water stable. Suitably, each test piece remains a single entity with no disintegration. The material may exhibit some swelling or other dimensional change and still be water stable. In a specific embodiment, the material does not exhibit a substantial decrease in dimension when subjected to the described water stability test. The term "substantial decrease in dimension" means that the average maximum axis length of the tests pieces exhibits more than a 15% decrease on average. In a more specific embodiment, the average maximum axis length of the test pieces exhibits no more than a 10% decrease on average. Averages are typically based on ten or more test pieces.

The present fibers, articles comprising fibers, and processes employ starch. In one embodiment, the invention is directed to fibers made from a thermoplastic starch composition comprising destructured starch, polyhydric alcohol, and acid and/or triglyceride; the fibers may be rendered water stable by heating. The thermoplastic polymer compositions of the present invention are made from mixtures of materials also referred to herein as "starch compositions".

### Starch

Starch is naturally abundant and can be relatively inexpensive. Thermoplastic starch can have desirable properties not typically observed in conventional petroleum-based polymers including, but not limited to, biodegradability, compostability, natural hydrophilicity and compatibility with materials traditionally incompatible with petroleum-based polymers.

Starch may take several different forms. As used herein, "native starch" means starch as it is found in its naturally occurring, unmodified form. Any suitable source of native starch is of use in the present invention. Non-limiting examples of sources include: corn starch, potato starch, sweet potato starch, wheat starch, sago palm starch, tapioca starch, rice starch, soybean starch, arrow root starch, bracken starch, lotus starch, cassava starch, waxy maize starch, high amylase corn starch, commercial amylase powder, and combinations thereof.

Native starch generally has a granular structure. In order to render starch capable of further processing, it is typically subject to a destructuring process. Without wishing to be bound by theory, it is believed that a starch granule is comprised of discrete amylopectin and amylase regions. To convert native starch to "destructured starch", the regions are broken apart during the destructurization process, which is often followed by a volume expansion of the starch, particularly in the presence of additives including, but not limited to, plasticizer. The presence of a plasticizer, such as polyhydric alcohol, when starch is destructured typically increases the starch's viscosity as compared to starch that is destructured in its absence. The destructuring process is typically irreversible. In some embodiments of the present invention, it may be desirable to destructure the starch as fully as possible, so as to avoid "lumps" which may have an adverse impact in subsequent processing steps including, but not limited to fiber spinning processes.

Native starch of use in the present invention may be destructured prior to its inclusion in the mixtures of present invention. In addition, or in the alternative, native starch may be destructured after it is in the mixture, i.e., *in situ.* In some embodiments of the present invention, the use of native starch is less expensive than using destructured starch, as it eliminates the use of a separate, destructuring step.

Native starch may be destructured using any suitable means. At least partial destructuring may be achieved through means including, but not limited to: heating; enzyme modification; chemical modification including but not limited to ethoxylation and the like (such as by adding ethylene oxide for example); chemical degradation; and combinations thereof. Agents that may act as starch plasticizers may be used to destructure the starch. In some embodiments, these agents may remain mixed with the starch during further processing. In other embodiments, the agents may be transient, meaning that they are removed so that they are not present during further processing, and/or in the final fiber or article comprising the fiber.

In some embodiments, destructured starch may encompass native starch that has been destructured by modification, as discussed above. Modified starch is defined as a native starch that has had its native molecular characteristics (molecular weight or chemical structure) altered in any way. For example, in some embodiments, if the molecular weight of the native starch is changed, but no other changes are made to the native starch, then the starch can be referred to as a modified starch. Chemical modifications of starch typically include acid or alkali hydrolysis and oxidative chain scission to reduce molecular weight and molecular weight distribution. Native starch generally has a very high average molecular weight and a broad molecular weight distribution (e.g. native corn starch has an average molecular weight of up to about 60,000,000 grams/mole (g/mol)). The average molecular weight of starch can be reduced as desired for the present invention by acid reduction, oxidation reduction, enzymatic reduction, hydrolysis (acid or alkaline catalyzed), physical/mechanical degradation (e.g., via the thermomechanical energy input of the processing equipment), and combinations thereof. The thermomechanical method and the oxidation method offer an additional advantage when carried out *in situ.* The exact chemical nature of the starch and molecular weight reduction method is not critical as long as the average molecular weight is in an acceptable range. Ranges of weight average molecular weight for starch or starch blends added to the melt can be from about 3,000 g/mol to about 8,000,000 g/mol, from about 10,000 g/mol to about 5,000,000 g/mol, or from about 20,000 g/mol to about 3,000,000 g/mol. In other embodiments, the average molecular weight is otherwise within the above ranges but about 1,000,000 or less, or about 700,000 or less. Starches having different molecular weights may be mixed as desired for use in the invention.

In some embodiments, destructured starch encompasses substituted starch. Substituted starches are starches that have some of their alcohol (i.e., hydroxyl) functional groups replaced by other chemical moieties. If substituted starch is desired, chemical modifications of starch typically include etherification and esterification. Chemical modification can be accomplished using ethylene oxide, otherwise known as ethoxylation, resulting in destructured starch as discussed above. Substituted starches may be desired for better compatibility or miscibility with the thermoplastic polymer and plasticizer. However, it may be desirable to balance substitution with the reduction in the rate of degradability. The degree of substitution of the chemically substituted starch is typically from about 1 % to about 100% (i.e., completely substituted). Alternatively, a low degree of substitution, from about 1% to about 6%, may be used.

In some embodiments, the starch compositions or the thermoplastic compositions of the present invention comprise from about 1% to about 99%, from about 30% to about 90%, from about 50% to about 85%, or from about 55% to 80% of starch, including the bound water content of the starch. The starch is selected from the group consisting of native starch, destructured starch (which may include modified starch and/or substituted starch) and combinations thereof. The term "bound water" refers to the water found naturally occurring in starch before it is mixed with other components to make the composition. In contrast, the term "free water" refers to water that may be added to a composition of the present invention. For example, free water may be incorporated as or with a plasticizer. A person of ordinary skill in the art will recognize that once the components are mixed in a composition, water can no longer be distinguished by its origin. Starch that has not been subjected to drying processes typically has bound water content under ambient conditions of about 5% to about 16% by weight of starch. In some embodiments of the present invention, the compositions and articles of the invention comprise at least about 50% destructured starch, more specifically, at least about 60% destructured starch.

Starch of use in the present invention may comprise any combination of starches as described generally or specifically herein, or as known in the art. Suitable starches of use may be selected from the group consisting of: cold water insoluble starch; cold water soluble starch; and combinations thereof. Wherein "cold water" refers to water that is at or below 25°C. As used herein, cold water insoluble starch is starch that dissolves less than 25% in water at 25° C.

Thermoplastic starch used herein refers to a starch composition that is capable of flowing when at an elevated temperature (significantly above normal ambient temperature; generally above 80°C), to the extent that the starch, or a composition comprising the starch, can be adequately processed, for example, for formation of homogeneous mixtures, spinning performance and/or desired fiber properties. The fibers and/or plastic articles comprising them are capable of solidifying after the elevated temperature is lowered to ambient temperatures to retain the shaped form.

### Polyhydric alcohol

"Polyhydric alcohol" as used herein refers to an alcohol having two or more alcohol (i.e., hydroxyl) functional groups. Without wishing to be bound by theory, it is believed (as mentioned above) that polyhydric alcohol may act as a starch plasticizer in the starch compositions of the present invention. In other words, polyhydric alcohol is believed to enable the starch to flow and to be processed, i.e., to create a thermoplastic starch.

Any suitable polyhydric alcohol or combination of polyhydric alcohols is of use. Non-limiting examples of suitable polyhydric alcohols include: glycerol (also known in the art as glycerin), glycol, sugar, sugar alcohol, and combinations thereof. Non-limiting examples of glycols of use include: ethylene glycol, propylene glycol, dipropylene glycol, butylene glycol, hexane triol, and the like, polymers thereof, and combinations thereof. Non-limiting examples of sugars of use include: glucose, sucrose, fructose, raffinose, maltodextrose, galactose, xylose, maltose, lactose, mannose, erythrose, pentaerythritol, and mixtures thereof. Non-limiting examples of sugar alcohols of use include: erythritol, xylitol, malitol, mannitol, sorbitol, and mixtures thereof. In specific embodiments of the present invention, the polyhydric alcohol comprises glycerol, mannitol, sorbitol, and combinations thereof.

In general, the polyhydric alcohol is substantially compatible with the polymeric components with which it is intermixed. As used herein, the term "substantially compatible" means that when heated to a temperature above the softening and/or the melting temperature of the composition, the polyhydric alcohol is capable of forming a visually homogeneous mixture with polymer present in the component in which it is intermixed. In some embodiments, the plasticizer is water soluble.

In some embodiments of the present invention, the polyhydric alcohol may also be used as a destructuring agent for starch. In these embodiments, upon destructuring the starch, the polyhydric alcohol may act as a plasticizer to the destructured starch, thereby rendering it thermoplastic. In further embodiments, upon destructuring the starch, the polyhydric alcohol may be removed and substituted with a different plasticizer to render the destructured starch thermoplastic. In some embodiments, the polyhydric alcohol may improve the flexibility of the resulting fibers and/or plastic articles comprising them.

Polyhydric alcohol is included in the present thermoplastic compositions in any suitable amount for either destructuring starch and/or rendering destructured starch thermoplastic. Generally, the amount of polyhydric alcohol needed is dependent upon the molecular weight of the starch, the amount of starch in the mixture, the affinity of the polyhydric alcohol for the starch, and combinations thereof. The polyhydric alcohol should sufficiently render the starch component thermoplastic so that it can be processed effectively, for example to form plastic articles. Generally, the amount of polyhydric alcohol increases with increasing molecular weight of starch. Typically, the polyhydric alcohol can be present in compositions of the present invention in an amount of from about 2% to about 70%, from about 5% to about 50%, from about 10% to 30%, or from about 15% to about 25%.

### Acid

Acids of use in the present invention have at least one functional group selected from the group consisting of: carboxylic acid, carboxylic acid anhydride, and combinations thereof. Such acids include, but are not limited to, monoacids, diacids, polyacids (acids having at least three acid groups), polymers comprising at least one acid moiety, co-polymers comprising at least one acid moiety, anhydrides thereof, and mixtures thereof.

Non-limiting examples of acids of use include: adipic acid, sebatic acid, lauric acid, stearic acid, myristic acid, palmitic acid, oleic acid, linoleic acid, sebacic acid, citric acid, oxalic acid, malonic acid, succinic acid, glutaric acid, maleic acid, fumaric acid, phthalic acid, isophthalic acid, terphthalic acid, acrylic acid, methacrylic acid, itaconic acid, glycidyl methacrylate, and combinations thereof. Anhydrides of such acids may also be employed within the context of the present invention. Non-limiting examples of acid anhydrides of use include: maleic anhydride, phthalic anhydride, succinic anhydride and combinations thereof.

Polymers and co-polymers comprising at least one acid moiety, and/or their anhydrides are of use. Suitable polymers and copolymers include, but are not limited to, those comprising monomer units of acrylic acid, methacrylic acid, itaconic acid, glycidyl methacrylate, anhydrides thereof, and combinations thereof. The polymer can contain other monomer units in conjunction with these acid monomer units. For example, ethylene-acid monomer copolymers such as ethylene-acrylic acid copolymer can be used. In a specific embodiment, the copolymers comprise at least 50 mol% of acid monomer units. The molecular weight of such polymers and copolymers can vary from as low as about 2,000 to over about 1,000,000. An example of a suitable polyacrylic acid is from Aldrich Chemical Company, having a molecular weight of about 450,000. An example of a suitable ethylene-acrylic acid copolymer is Primacore 59801 from Dow Chemical, having an acrylic acid content of at least 50 mol%.

In specific embodiments, the acid comprises at least one diacid, polyacid, acid polymer or copolymer, or a mixture thereof. In other embodiments, the acid comprises a diacid, alone or in combination with another acid, for example a monoacid. In further embodiments, the acid comprises adipic acid, stearic acid, lauric acid, citric acid, polyacrylic acid and/or ethylene-acrylic acid copolymer.

Typically, the acid is employed in the starch composition in an amount of from about 0.1% to about 30%, from about 1% to about 20%, or from about 2% to about 12%. In some embodiments, the molar ratio of alcohol functional groups to acidic functional groups in the starch composition is at least about 1:1, or at least about 4:1. In some embodiments, the molar ratio of alcohol functional groups to acidic groups in the starch composition is from about 1:1 to about 200:1, or from about 1:1 to about 50:1.

### Triglyceride

Any suitable triglycerides, which are also known in the art as triacylglycerols, are of use in the present invention. Non-limiting examples of triglycerides of use include: tristearin, triolein, tripalmitin, 1,2-dipalmitoolein, 1,3-dipalmitoolein, 1-palmito-3-stearo-2-olein, 1-palmito-2-stearo-3-olein, 2-palmito-1-stearo-3-olein, trilinolein, 1,2-dipalmitolinolein, 1-palmito-dilinolein, 1-stearo-dilinolein, 1,2-diacetopalmitin, 1,2-distearo-olein, 1,3-distearo-olein, trimyristin, trilaurin and combinations thereof.

Suitable triglycerides may be added to the present compositions in neat form. Additionally, or alternatively, oils and/or processed oils containing suitable triglycerides may be added to the compositions. Non-limiting examples of oils include coconut oil, corn germ oil, olive oil, palm seed oil, cottonseed oil, palm oil, rapeseed oil, sunflower oil, whale oil, soybean oil, peanut oil, linseed oil, tall oil, and combinations thereof.

Typically, triglycerides are employed in the starch compositions in an amount of from about 0.1% to about 30%, from about 1 % to about 20%, or from about 2% to about 12%. In some embodiments, the molar ratio of alcohol functional groups to ester functional groups in the starch composition is at least about 1:1, or at least about 4:1. In some embodiments, the molar ratio of alcohol functional groups to ester functional groups in the starch composition is from about 1:1 to about 200:1, or from about 1:1 to about 50:1.

In some embodiments, combinations of acid and triglyceride are employed in the starch compositions. In some embodiments, the total amounts of acid and triglyceride is from about 0.1% to about 32%, from about 1% to about 25%, or from about 2% to about 20%. Additionally, or alternatively, the molar ratio of the alcohol functional groups to the total of ester and acid functional groups is at least about 1:1, or at least about 4:1. In some embodiments, the molar is from about 1:1 to about 200:1, or from about 1:1 to about 50:1.

### Additional components

The compositions according to the present invention may include one or more additional components as desired for the processing and/or end use of the fibers and or plastic articles. Additional components may be present in any suitable amount. In some embodiments, additional components may be present in an amount of from about 0.01% to about 35% or from about 2% to about 20%. Non-limiting examples of additional components include, but are not limited to, additional polymers, processing aids and the like.

Non-limiting examples of additional polymers of use include: polyhydroxyalkanoates, polyvinyl alcohol, polyethylene, polypropylene, polyethylene terephthalate, maleated polyethylene, maleated polypropylene, polylactic acid, modified polypropylene, nylon, caprolactone, and combinations thereof.

In embodiments in which properties including, but not limited to, biodegradability and/or flushability are desired, additional suitable biodegradable polymers and combinations of thereof are of use. In some embodiments, polyesters containing aliphatic components are suitable biodegradable thermoplastic polymers. In some embodiments, among the polyesters, ester polycondensates containing aliphatic constituents and poly(hydroxycarboxylic) acid are preferred. The ester polycondensates include, but are not limited to: diacids/diol aliphatic polyesters such as polybutylene succinate, and polybutylene , succinate co-adipate; aliphatic/aromatic polyesters such as terpolymers made of butylenes diol, adipic acid, and terephtalic acid. The poly(hydroxycarboxylic) acids include, but are not limited to: lactic acid based homopolymers and copolymers; polyhydroxybutyrate; and other polyhydroxyalkanoate homopolymers and copolymers. In some embodiments, a homopolymer or copolymer of poly lactic acid is preferred. Modified polylactic acid and different stereo configurations thereof may also be used. Suitable polylactic acids typically have a molecular weight range of from about 4,000 g/mol to about 400,000 g/mol . Examples of suitable commercially available poly lactic acids include NATUREWORKS^{™} from Cargill Dow and LACEA^{™} from Mitsui Chemical. An example of a suitable commercially available diacid/diol aliphatic polyester is the polybutylene succinate/adipate copolymers sold as BIONOLLE^{™} 1000 and BIONOLLE^{™} 3000 from the Showa Highpolymer Company, Ltd. Located in Tokyo, Japan. An example of a suitable commercially available aliphatic/aromatic copolyester is the poly(tetramethylene adipate-co-terephthalate) sold as EASTAR BIO^{™} Copolyester from Eastman Chemical or ECOFLEX^{™} from BASF. In some embodiments, the biodegradable polymer or combination of polymers may comprise polyvinyl alcohol.

The aforementioned biodegradable polymers and combinations thereof are present in an amount will be from about 0.1% to about 70%%, from about 1 % to about 50%, or from about 2% to about 25%, by weight of the present starch and thermoplastic starch compositions.

Processing aids are generally present in the current compositions in amounts of from about 0.1% to about 3%, or from about 0.2% to about 2%. Non-limiting examples of processing aids include: lubricants, anti-tack, polymers, surfactants, oils, slip agents, and combinations thereof. Non-limiting examples of specific processing aids include: Magnesium stearate; fatty acid amides; metal salts of fatty acids; wax acid esters and their soaps; montan wax acids, esters and their soaps; polyolefin waxes; non polar polyolefin waxes; natural and synthetic paraffin waxes; fluoro polymers; talc; silicon; clay; diatomaceous earth. Commercial examples of such compounds include, but are not limited to: Crodamide^{™} (Croda, North Humberside, UK), Atmer™ (Uniqema, Everberg, Belgium,) and Epostan™ (Nippon Shokobai, Tokyo, JP).

In some embodiments, the starch comprises at least about 50% of all polymer components in the starch compositions, more specifically at least about 60% of all polymer components in the starch compositions.

### Water Stability

Without wishing to be bound by theory, the thermoplastic polymer compositions according to the present invention may be rendered water stable via the aforementioned ester condensation reaction and/or transesterification reaction. When the thermoplastic polymer compositions are made into fibers and/or articles comprising fibers, the reactions may be induced before formation of the fiber and/or article, during formation of the fiber and/or article, after the fiber's and/or article's formation (i.e., curing) and combinations thereof. In some embodiments, the reaction(s) are induced, and/or driven towards completion through the application of heat. In some embodiments of the present invention, a catalyst may be used to initiate and/or accelerate the ester condensation and/or transesterification reactions. Any suitable catalyst is of use. Non-limiting examples of useful catalysts include Lewis acids. A non-limiting example of a Lewis acid is para-toluene sulfonic acid.

With regard to the ester condensation reaction, it is believed without being bound by theory that the heating of the thermoplastic polymer composition comprising acid, may remove a sufficient amount of water from the starch composition, (including some, but not all of the bound water) to allow a reaction of the polyhydric alcohol and the acid to form a water stable reaction product to an extent that provides the resulting composition with water stability. While again not wishing to be bound by theory, it is believed that a condensation reaction may occur between the polyhydric alcohol and acid. Generally, the chemistry which governs such condensation reactions is known in the art as alkyd chemistry.

In the present invention, it may be important that the ester condensation reaction is not completed to such an extent that a gel of the reaction products is formed before final processing of the thermoplastic composition occurs. As used herein "gel" means a material that is crosslinked to an extent that flow even under high temperatures is no longer possible without degradation of the material's molecular weight. It is important for the system to be below the gel point of the reactants before final processing so as to retain sufficient flow behavior to enable shaping the material into films fibers or articles. The gel point is defined as the state at which enough polymer chains formed by the products of the reactants are bonded together such that at least one very large molecule is coextensive with the polymer phase and flow is no longer possible and the material behaves more like a solid.

Up until to the gel point, it may be advantageous for the reaction to proceed to a point where prepolymers such as oligomers or even larger molecules are formed, yet these species should retain the ability to flow and be shaped into useful articles. Oligomers as used herein are reaction products from constituent monomers that include at least two monomers up to about ten monomers. In some embodiments of the current invention, when carrying out the ester condensation reaction between the acid and alcohol and thereby forming oligomers, it may be advantageous to remove excess water from the reaction product before forming the end product. It is believed that removal of the water will speed the ester condensation reaction toward completion in the final processing step.

In some embodiments, the thermoplastic composition is heated at a temperature of at least about 90°C, more specifically at least about 100°C, to convert the thermoplastic composition to a water stable composition. Typically, the thermoplastic composition will not be heated at a temperature over about 250°C, or over about 225°C. In some embodiments, the thermoplastic composition is heated at a temperature of at least about 115°C to convert the thermoplastic composition to a water stable composition. In further embodiments, the thermoplastic composition is heated at a temperature of from about 130°C to about 180°C to convert the thermoplastic composition to a water stable composition. In some embodiments, the water content of the composition is reduced to a level below the level of bound water naturally present in the starch at ambient conditions. In other embodiments, the water content of the composition is reduced to 5% or less of the composition. In other embodiments, water content is about 4% or less. In another embodiment the water content is reduced to about 3% or less. In yet another embodiment, the water content is reduced to about 2% or less. Water content can be reduced by providing the starch composition at elevated temperatures under conditions wherein water can vaporize.

Although not required, the physical form of the thermoplastic polymer composition may be modified to provide a greater surface area to facilitate water removal from the compositions. The heating time necessary to convert a thermoplastic composition to a water stable form will depend, in general, on a variety of factors, including component compositions (i.e., particular starch, polyhydric alcohol and acid and/or triglyceride), heating temperature, physical form of the composition, and the like. Suitable times may range from instantaneously to about 24 hours, about 1 minute to about 24 hours, from about 5 minutes to about 12 hours, or from about 5 minutes to about 1 hour. In general, water content should not be reduced under conditions wherein decomposition, burning or scorching of the starch occurs, particularly in the case that visually noticeable or significant levels of decomposition, burning or scorching occurs.

In some embodiments, the thermoplastic compositions according to the present invention are formed by melt mixing and/or extruding a mixture comprising destructured starch, polyhydric alcohol, and acid and/or triglyceride, using conventional mixing and/or extrusion techniques. The mixture may be formed by combining destructured starch, polyhydric alcohol, and acid and/or triglyceride. Alternatively, the mixture may be provided by combining non-destructured starch, polyhydric alcohol, and acid and/or triglyceride, with the additional step of destructuring the starch *in situ* in the mixture, by any of the destructuring techniques discussed above. The components are typically mixed using conventional compounding techniques. The objective of the compounding step is to produce at least a visually homogeneous melt composition comprising the starch.

A suitable mixing device is a multiple mixing zone twin screw extruder with multiple injection points. The multiple injection points can be used to add the destructured starch, polyhydric alcohol and acid and/or triglyceride. A twin screw batch mixer or a single screw extrusion system can also be used. As long as sufficient mixing and heating occurs, the particular equipment used is not critical. An alternative method for compounding the materials comprises adding the starch, polyhydric alcohol, and acid and/or triglyceride to an extrusion system where they are mixed in progressively increasing temperatures. For example, a twin screw extruder with six heating zones may be employed. This procedure can result in minimal thermal degradation of the starch and may ensure that the starch is fully destructured. However, it may not be necessary to extrude a melt mixture, and, in general, any method known in the art or suitable for the purposes hereof can be used to combine the ingredients of the components to form the thermoplastic compositions of the present invention. Typically such techniques will include heat and mixing, and optionally pressure. The particular order or mixing, temperatures, mixing speeds or time, and equipment can be varied, as will be understood by those skilled in the art, however temperature should be controlled such that the starch does not significantly degrade. Further, if the temperature of the melt mixing and/or extrusion process is sufficiently high and for a sufficient time to eliminate at least a portion of bound water from the starch and drive a reaction between the polyhydric alcohol and the acid, the thermoplastic composition which is formed by melt extruding these components will convert to a water stable composition. For example, the melt extrusion can be conducted in an extruder provided with vents or other modifications which facilitate water removal and the conversion to a water stable composition. In such an embodiment, it is therefore advantageous to melt extrude the composition to a form which is suitable for and end use including, but not limited to, fibers or nonwovens comprising the fibers.

On the other hand, if the temperature or conditions at which the melt extrusion of the mixture comprising destructured starch, polyhydric alcohol, acid and/or triglyceride is conducted at a sufficiently low temperature and/or for an insufficient time to eliminate at least a portion of bound water from the starch and drive reaction between the polyhydric alcohol, acid and/or triglyceride, the resulting extrudate comprises thermoplastic compositions of the invention, which may be further processed, if desired, and which are convertible to water stable compositions by further heating. The extrudate can therefore be provided in this embodiment in a form which facilitates handling, further processing, or the like. For example, a thermoplastic composition extrudate can be in pellet form, powder or crumb form or the like. In a specific embodiment, the thermoplastic composition extrudate is in a pellet form which is then suitable for melt extruding to a desired end use form. In this embodiment, the further melt extrusion of pellets (or extrudate of another form) to form fibers, or articles comprising fibers, may be conducted under sufficient conditions of temperature and time to effect the conversion of the thermoplastic composition to a water stable composition or article. Alternatively, if the melt extrusion is not conducted under sufficient conditions of temperature and time to effect the conversion of the thermoplastic composition to a water stable composition, the resulting extrudate may be heated further to effect the conversion of the extruded thermoplastic composition to a water stable article.

In some embodiments, a thermoplastic composition in the form of pellets is formed by melt extruding destructured starch, polyhydric alcohol and acid and/or triglyceride. The extrusion process may not provide sufficient heating of the thermoplastic composition for a sufficient time to effect conversion to a water stable composition. The pellets are subsequently subjected to melt extrusion by conventional fiber spinning processes. The resulting fibers are rendered water stable by an additional heating step at a temperature of from about 100°C, more specifically 115°C, still more specifically from about 130°C, to about 180°C. Alternatively, the melt spinning process is conducted at a temperature in this range under conditions by which the resulting fibers are rendered water stable. In a further embodiment, the necessary water is eliminated from the fibers by flash evaporation as the fibers exit the spinneret swing to the reduction in pressure.

In some embodiments, it may be advantageous to provide the polyhydric alcohol and the acid and/or triglyceride as what is termed herein as a "pre-polymer". In these instances, the aforementioned condensation reaction and/or transesterification reaction has already at least partially, but not completely, taken place between the polyhydric alcohol and the acid and/or triglyceride before it is mixed with the starch. In further embodiments, the pre-polymer may also contain starch. Pre-polymers may take any suitable form which may be convenient to make, ship process and combinations thereof. Non-limiting examples of forms include strands, pellets, powder, and combinations thereof.

In some embodiments, a thermoplastic composition in the form of pellets is formed by melt extruding destructured starch, polyhydric alcohol and acid and/or triglyceride. The extrusion process does not provide sufficient heating of the thermoplastic composition for a sufficient time to effect conversion to a water stable composition. The pellets are subsequently subjected to melt extrusion by conventional fiber spinning processes. The resulting fibers are rendered water stable by an additional heating step at a temperature of from about 100°C, more specifically 115°C, still more specifically from about 130°C, to about 180°C. Alternatively, the melt spinning process is conducted at a temperature in this range under conditions by which the resulting fibers are rendered water stable. In a further embodiment, the necessary water is eliminated from the fibers by flash evaporation as the fibers exit the spinneret and are subject to a reduction in pressure.

In general, high fiber spinning rates are desired. Fiber spinning speeds of about 10 meters/minute or greater can be used. In some embodiments hereof, the fiber spinning speed is from about 100 to about 7,000 meters/minute, or from about 300 to about 3,000 meters/minute, or from about 500 to about 2,000 meters/minute. The spun fibers can be collected using conventional godet winding systems or through air drag attenuation devices. If the godet system is used, the fibers can be further oriented through post extrusion drawing as desired. The drawn fibers may then be crimped and/or cut to form non-continuous fibers (staple fibers) used in a carding, airlaid, or fluidlaid process. The fiber may be made by fiber spinning processes using a high draw down ratio. The draw down ratio is defined as the ratio of the fiber at its maximum diameter (which is typically occurs immediately after exiting the capillary of the spinneret in a conventional spinning process) to the final diameter of the formed fiber. The fiber draw down ratio via either staple, spunbond, or meltblown process will typically be 1.5 or greater, and can be about 5 or greater, about 10 or greater, or about 12 or greater. Continuous fibers can be produced through, for example, spunbond methods or meltblowing processes. Alternately, non-continuous (staple fibers) fibers can be produced according to conventional staple fiber processes as are well known in the art. The various methods of fiber manufacturing can also be combined to produce a combination technique, as will be understood by those skilled in the art. Additionally, hollow core fibers as disclosed in U.S. Patent No. 6,368,990 can be formed.

Typically, the diameter of fibers produced according to the present invention is less than about 200 microns, and in alternate embodiments is less than about 100 microns, less than about 50 microns, or less than about 30 microns. In one embodiment, the fibers have a diameter of from about 0.1 microns to about 25 microns. In another embodiment the fibers may have a diameter from about 0.2 microns to about 15 microns. In other embodiment, the fibers may have a diameter from about 5 microns to about 14 microns. Fiber diameter is controlled by factors well known in the fiber spinning art including, for example, spinning speed and mass throughput.

Fibers according to the present invention include, but are not limited to, monocomponent fibers, multicomponent fibers (such as bicomponent fibers), or biconstituent fibers. The fibers may take any suitable shape including, round or non-round. Non-round fibers include, but are not limited to those described above.

In some embodiments, the fiber is a multicomponent fiber having a sheath and a core. Either the core or the sheath or both the core and sheath may comprise a thermoplastic starch composition according to the present invention. In embodiments, in which the core is a thermoplastic composition according to the present invention, the sheath comprises a different polymer. Non-limiting examples of such polymers include those selected from the group consisting of: polyethylene terephthalate; polyethylene; polypropylene; polyhydroxyalkanoate; polylactic acid; polyester; and combinations thereof. In embodiments in which the fiber is a multicomponent fiber having an islands-in-the-sea configuration, wherein either the islands, the sea or both comprise a thermoplastic starch composition according to the present invention. In embodiments, in which the islands are a thermoplastic composition according to the present invention, the sea comprises a different polymer. Non-limiting examples of such polymers include those selected from the group consisting of: polyethylene terephthalate; polyethylene; polypropylene; polyhydroxyalkanoate; polylactic acid; polyester; and combinations thereof.

The fibers according to the present invention may be used for any purposes for which fibers are conventionally used. This includes, without limitation, incorporation into nonwoven webs and substrates. The fibers hereof may be converted to nonwovens by any suitable methods known in the art. Continuous fibers can be formed into a web using industry standard spunbond type technologies while staple fibers can be formed into a web using industry standard carding, airlaid, or wetlaid technologies. Typical bonding methods include: calendar (pressure and heat), thru-air heat, mechanical entanglement, hydrodynamic entanglement, needle punching, and chemical bonding and/or resin bonding. The calendar, thru-air heat, and chemical bonding are the preferred bonding methods for the starch and polymer multicomponent fibers. Thermally bondable fibers are required for the pressurized heat and thru-air heat bonding methods.

The fibers of the present invention may also be bonded or combined with other synthetic or natural fibers to make nonwoven articles. The synthetic or natural fibers may be blended together in the forming process or used in discrete layers. Suitable synthetic fibers include fibers made from polypropylene, polyethylene, polyester, polyacrylates, and copolymers thereof and mixtures thereof. Natural fibers include cellulosic fibers and derivatives thereof. Suitable cellulosic fibers include those derived from any tree or vegetation, including hardwood fibers, softwood fibers, hemp, and cotton. Also included are fibers made from processed natural cellulosic resources such as rayon.

The fibers described herein are typically used to make disposable nonwoven materials for use in articles which may find applications in one of many different uses. Specific articles of the present invention include disposable nonwovens for hygiene and medical applications, more specifically, for example, in applications such as diapers, wipes, feminine hygiene articles, drapes, gowns, sheeting, bandages and the like. In diapers, nonwoven materials are often employed in the top sheet or back sheet, and in feminine pads or products, nonwoven materials are often employed in the top sheet. Nonwoven articles generally contain greater than about 15 % of a plurality of fibers that are continuous or non-continuous and physically and/or chemically attached to one another. The nonwoven may be combined with additional nonwovens or films to produce a layered article used either by itself or as a component in a complex combination of other materials. Nonwoven articles produced from fibers can also exhibit desirable mechanical properties, particularly, strength, flexibility and softness. Measures of strength include dry and/or wet tensile strength. Flexibility is related to stiffness and can attribute to softness. Softness is generally described as a physiologically perceived attribute which is related to both flexibility and texture. One skilled in the art will appreciate that the fibers according to the invention are also suitable for use in applications other than nonwoven articles.

Notwithstanding the water stability of the fibers and other articles produced in the present invention, the articles may be environmentally degradable depending upon the amount of starch that is present, any additional polymer used, and the specific configuration of the article. "Environmentally degradable" is defined as being biodegradable, disintegratable, dispersible, flushable, or compostable or a combination thereof. In the present invention, the fibers, nonwoven webs, and articles may be environmentally degradable.

A specific embodiment of a method according to the invention is described. A starch is destructured by ethoxylation, and a polyhydric alcohol, such as glycerol, is added to the destructured starch. A liquid polyhydric alcohol such as glycerol can be combined with destructured starch via a volumetric displacement pump. The starch and polyhydric alcohol mixture is added to a mixer and typically heated to at least 100°C over a period of from about 1 to 5 minutes at about 60 rpm. Acid is added to the mixer, with continued heating over a period of from about 1 to about 15 minutes at about 60 rpm. Alternatively, multiple feed zones can be used for introducing starch, polyhydric alcohol, and acid, or premixtures thereof, directly to an extruder. The resulting mixture of starch, polyhydric alcohol and acid is extruded as a rod and chopped into pellets using any suitable cutting device including, but not limited to, a knife. After from about 18 to about 36 hours, the pellets are placed in an extruder. The extruder barrel is preheated to at temperature of about 100°C to about 200°C. Fibers are extruded by melt spinning at a temperature sufficient to flash off residual water and render the fibers water stable.

The starch-containing compositions and process of the present invention can also be used to make forms other than fibers, such as, but not limited to, films and molded articles using conventional techniques known in the art.

### EXAMPLES

The examples below further illustrate the present invention.

### Example 1

This example demonstrates melt mixing and one-shot spinning of water stable fibers. The following materials are mixed in a Haake Rheocord 90 melt mixer, Thermo Electron Corporation, Newington, NH:
30 g Ethylex^{™} 2015 hydroxyethylated starch (Tate& Lyle, Decatur, IL)
12.5 g Glycerol (Aldrich Chemicals, St. Louis, MO)
7.5 g Stearic Acid (Aldrich Chemicals, St. Louis, MO)
7.5 g Adipic Acid (Aldrich Chemicals, St. Louis, MO)

The starch and the glycerol are mixed for about 3 minutes at about 60 rpm at a temperature of about 160°C. The balance of components is added and mixed for an additional 7 minutes at about 60 rpm. The contents are removed and allowed to cool to room temperature. The mixture is then chopped using a knife into pieces approximately 50 mm in diameter.

After 24 hours, the pieces are placed into a piston/cylinder one shot spinning system, Alex James, Inc. of Greer, SC. The extruder barrel is preheated to 160°C. The spinneret capillary is 0.016" diameter and has an L/D of 3. Fibers are extruded by activating the piston at an extrusion rate of approximately 0.8 g/minute. Approximately 50 g of fibers are collected.

Approximately 20 g of the fibers are dried in a vacuum oven at 90°C and 30 mm Hg for 12 hours. Another 20 g of the fibers are dried in a convection oven at 115°C for 12 hours. The remaining 10 g of fibers are simply allowed to cool for 12 hours at ambient air temperature (about 22°C). The respective fibers are subjected to the water stability test as described herein. The fibers which are dried at elevated temperature (90°C and 115°C) do not dissolve or breakup, displaying water stability as defined herein. Fibers that are allowed simply to cool, without heat treatment, break up completely after 1 hour in water.

### Comparative Example 2

This example demonstrates a conventional process for melt mixing and one-shot spinning of starch fibers which are not water stable. The following materials are mixed in the described Haake Rheocord 90 melt mixer:
30 g Ethylex™ 2015 starch (Tate& Lyle, Decatur, IL)
12.5 g Glycerol (Aldrich Chemicals, St. Louis, MO)

The starch and the glycerol are mixed for about 10 minutes at about 60 rpm at a temperature of about 160°C. The contents are removed and allowed to cool to room temperature. The mixture is then chopped using a knife into pieces approximately 50 mm in diameter. After 24 hours, the pieces are placed into the described piston/cylinder one shot spinning system. The extruder barrel is preheated to 160°C. The spinneret capillary is 0.016" diameter and has an L/D of 3. Fibers are extruded by activating the piston at an extrusion rate of approximately 0.8 g/minute. Approximately 40 g of fibers are collected.

Approximately 10 g of the fibers are dried in a vacuum oven at 90°C and 30 mm Hg for 12 hours. Another 10 g of the fibers are dried in a convection oven at 115°C for 12 hours. The remaining 10 g of fibers are simply allowed to cool for 12 hours at ambient air temperature (about 22°C). The fibers are subjected to the described water stability test. In this case, the fibers that are dried at elevated temperature (90°C and 115°C) and those that are allowed to cool to ambient temperature all break up completely after 1 hour in water.

### Example 3

This example demonstrates melt mixing and one-shot spinning of water stable starch fibers of various compositions. The following materials are mixed in the described Haake Rheocord 90 melt mixer in a manner as described in Example 1 and melt blended. Approximately 50 g of each composition is made.

| Material, wt % | Ethylex™ 2015 starch (Tate& Lyle, Decatur, IL) | Glycerol (Aldrich Chemicals, St. Louis, MO) | Lauric Acid (Aldrich Chemicals, St. Louis, MO) | Adipic Acid (Aldrich Chemicals, St. Louis, MO) |
|---|---|---|---|---|
| Sample 1 | 60 | 25 | 12.5 | 2.5 |
| Sample 2 | 60 | 25 | 10 | 5 |
| Sample 3 | 60 | 25 | 7.5 | 7.5 |

After 24 hours, the materials are spun into fibers using the described piston/cylinder one shot spinning system. The extruder barrel is preheated to 160°C. The spinneret capillary is 0.016" diameter and has an L/D of 3. Fibers are extruded by activating the piston at an extrusion rate of approximately 0.8 g/minute. Approximately 40 g of fibers of each composition are collected.

Approximately 20 g of each composition of fibers are dried in a convection oven at 115°C for 12 hours, and about 10 g of each composition of fibers are simply allowed to cool for 12 hours at ambient air temperature (about 22°C). The fibers are subjected to the described water stability test, with the following results:

| Material | Result of water stability test for heat treated fibers (2 weeks) | Result of water stability test for untreated fibers (2 weeks) |
|---|---|---|
| Sample 1 | Pass | Fail |
| Sample 2 | Pass | Fail |
| Sample 3 | Pass | Fail |

### Example 4

This example demonstrates additional blending and spinning of fibers with water stability. The following materials are used:
3500 g Ethylex™ 2015 (Tate& Lyle, Decatur, IL)
1095 g Glycerol (Aldrich Chemicals, St. Louis, MO)
438 g Adipic acid (Solutia Chemicals, St. Louis, MO)
438 g Stearic acid (Aldrich Chemicals, St. Louis, MO)
50 g Magnesium stearate (Aldrich Chemicals, St. Louis, MO)

The starch, adipic acid, stearic acid and magnesium stearate (employed as a process aid) are dry mixed in a Henschel Raw Material Mixer (Green Bay, WI) for 4 minutes at 1000 rpm. The mixture is then fed into a B&P Process System Twin Screw Extrusion Compounding System (Saginaw, MI) with 40 mm co-rotating screws. Glycerol is fed through a liquid feed port at a rate that maintains the desired composition stated above. The screw speed is set at 90 rpm with the thermal profile as shown below:

| Temperature | zone 1 | zone 2 | zone 3 | zone 4 | zone 5 | zone 6 | zone 7 | zone 8 | zone 9 | die |
|---|---|---|---|---|---|---|---|---|---|---|
| Set(°C) | 85 | 85 | 100 | 145 | 155 | 160 | 160 | 160 | 140 | 100 |
| Actual (°C) | 83 | 83 | 85 | 138 | 138 | 144 | 155 | 147 | 133 | 98 |

At these conditions the overall extrusion rate is 20 lbs/hour. A vacuum line is applied to two of three vent ports to extract water from the material during pelletization. Torque is 10%. The mixture is extruded into strands 0.3-0.8 cm in diameter and the strands are chopped to form pellets via a Conair pellitizer. The pellets are dried for 12 hours in a through air dryer at 150°F. The pellets are fed into a Hills 4-hole extruder test stand (Hills, Inc., West Melbourne, FL) with a Hills bicomponent sheath/core 4-hole spin pack. The equipment features two extruders that feed to a single spin head to produce bicomponent fibers. For single component fibers, both extruders are set to identical conditions as follows and the same material is fed into both extruders:

| | Extruder Melt Pressure (psi) | Barrel Zone 1 (°C) | Barrel Zone 2 (°C) | Barrel Zone 3 (°C) | Extruder Pressure (psi) | Melt Pump Speed (rpm) | Spin Head (°C) |
|---|---|---|---|---|---|---|---|
| Set Extruder 1 | 1400 | 160 | 160 | 160 | 1500 | 464 | 165 |
| Set Extruder 2 | 1400 | 160 | 160 | 160 | 1500 | 464 | |

Fibers are collected in free fall at a mass throughput of 0.8 g/hole-min. The fibers are collected and dried overnight in a convection oven at 115°C . The fibers are subjected to the water stability test. All fibers pass the water stability test.

### Example 5'

This example demonstrates blending and spinning of bicomponent fibers with water stability. The following materials are used to produce a thermoplastic composition:
3500 g Ethylex™ 2015 (Tate& Lyle, Decatur, IL)
1095 g Glycerol (Aldrich Chemicals, St. Louis, MO)
438 g Adipic acid (Solutia Chemicals, St. Louis, MO)
438 g Stearic acid (Aldrich Chemicals, St. Louis, MO)
50 g Magnesium stearate (Aldrich Chemicals, St. Louis, MO)

The starch , adipic acid, stearic acid and magnesium stearate are dry mixed in a Henschel Raw Material Mixer (Green Bay, WI) for 4 minutes at 1000 rpm. The mixture is then fed into the described B&P Process System Twin Screw Extrusion Compounding System. Glycerol is fed through a liquid feed port at a rate that maintains the desired composition stated above. The screw speed is set at 90 rpm with the thermal profile as employed in Example 4.

The overall extrusion rate is 20 lbs/hour. A vacuum line is applied to two of three vent ports to extract water from the material during pelletization. Torque is 10%. The mixture is extruded into strands 0.3-0.8 cm in diameter and the strands are chopped to form pellets via a Conair pellitizer. The pellets are dried for 12 hours in a through air dryer at 150°F. The pellets are fed in the described Hills 4-hole extruder test stand with the bicomponent sheath/core 4-hole spin pack. For bicomponent fibers, the thermoplastic composition as described above is fed into extruder 1. In the second extruder a polylactic acid (PLA) obtained from Natureworks LLC (Grade 6251D) is used, under the following conditions:

| | Extruder Melt Pressure (psi) | Barrel Zone 1 (°C) | Barrel Zone 2 (°C) | Barrel Zone 3 (°C) | Extruder Pressure (psi) | Melt Pump Speed (rpm) | Spin Head (°C) |
|---|---|---|---|---|---|---|---|
| Set Extruder 1 (TPS) | 1400 | 180 | 190 | 190 | 1500 | 464 | 190 |
| Set Extruder 2 (PLA) | 1400 | 150 | 160 | 160 | 1500 | 464 | |

This produces a 50/50 sheath/core fiber. The fibers are collected in free fall at a mass throughput of 0.8 g/hole-min. The fibers are dried overnight in a convection oven at 115°C. The fibers are subjected to the water stability test. All fibers passed.

### Example 6

### Fibers blended with PP

This example demonstrates additional blending and spinning of fibers with water stability. The following materials are used:
6000 g Ethylex™ 2065 (Tate& Lyle, Decatur, IL)
2500 g Glycerol (Aldrich Chemicals, St. Louis, MO)
500g Polypropylene Profax™ PH835 (Basell, Elkton, MD)
500g Maleated Polypropylene G3003 (Eastman Chemicals, Kingsport, TN)
500 g Adipic acid (Solutia Chemicals, St. Louis, MO)
50 g Magnesium stearate (Aldrich Chemicals, St. Louis, MO)

The solid components are dry mixed in a Henschel Raw Material Mixer (Green Bay, WI) for 4 minutes at 1000 rpm. The mixture is then fed into a B&P Process System Twin Screw Extrusion Compounding System (Saginaw, MI) with 40 mm co-rotating screws. Glycerol is fed through a liquid feed port at a rate that maintains the desired composition (stated above). The screw speed is set at 90 rpm with the thermal profile as shown below:

| Temperature | zone 1 | zone 2 | zone 3 | zone 4 | zone 5 | zone 6 | zone 7 | zone 8 | zone 9 | die |
|---|---|---|---|---|---|---|---|---|---|---|
| Set(°C) | 85 | 85 | 100 | 145 | 155 | 160 | 160 | 160 | 140 | 100 |
| Actual (°C) | 83 | 83 | 85 | 138 | 138 | 144 | 155 | 147 | 133 | 98 |

At these conditions the overall extrusion rate is 20 lbs/hour. A vacuum line is applied to two of three vent ports to extract water from the material during pelletization. Torque is 10%. The mixture is extruded into strands 0.3-0.8 cm in diameter and the strands are chopped to form pellets via a Conair pellitizer. The pellets are dried for 12 hours in a through air dryer at 150°F. The pellets are fed into a Hills 4-hole extruder test stand (Hills, Inc., West Melbourne, FL) with a Hills bicomponent sheath/core 4-hole spin pack. The equipment features two extruders that feed to a single spin head to produce bicomponent fibers. For single component fibers, both extruders are set to identical conditions as follows and the same material is fed into both extruders:

| | Extruder Melt Pressure (psi) | Barrel Zone 1 (°C) | Barrel Zone 2 (°C) | Barrel Zone 3 (°C) | Extruder Pressure (psi) | Melt Pump Speed (rpm) | Spin Head (°C) |
|---|---|---|---|---|---|---|---|
| Set Extruder 1 (°C) | 1400 | 125 | 160 | 170 | 1500 | 464 | 175 |
| Set Extruder 2 (°C) | 1400 | 125 | 160 | 170 | 1500 | 464 | |

Fibers are collected through an attenuating air jet set at 20 psi. A mass throughput of 0.75 g/hole-min is maintained. The fibers are collected and dried overnight in a convection oven at 115°C . The fibers are subjected to the water stability test. All fibers pass the water stability test.

### Example 7

This example demonstrates blending and spinning of bicomponent fibers with water stability. The following materials are used to produce a thermoplastic composition:
6000 g Ethylex™ 2065 (Tate& Lyle, Decatur, IL)
2500 g Glycerol (Aldrich Chemicals, St. Louis, MO)
500g Polypropylene Profax™ PH835 (Basell, Elkton, MD)
500g Maleated Polypropylene G3003 (Eastman Chemicals, Kingsport, TN)
500 g Adipic acid (Solutia Chemicals, St. Louis, MO)
50 g Magnesium stearate (Aldrich Chemicals, St. Louis, MO)

The solid components are dry mixed in a Henschel Raw Material Mixer (Green Bay, WI) for 4 minutes at 1000 rpm. The mixture is then fed into a B&P Process System Twin Screw Extrusion Compounding System (Saginaw, MI) with 40 mm co-rotating screws. Glycerol is fed through a liquid feed port at a rate that maintains the desired composition (stated above). The screw speed is set at 90 rpm with the thermal profile as shown below:

| Temperature | zone 1 | zone 2 | zone 3 | zone 4 | zone 5 | zone 6 | zone 7 | zone 8 | zone 9 | die |
|---|---|---|---|---|---|---|---|---|---|---|
| Set (°C) | 85 | 85 | 100 | 145 | 155 | 160 | 160 | 160 | 140 | 100 |
| Actual (°C) | 83 | 83 | 85 | 138 | 138 | 144 | 155 | 147 | 133 | 98 |

At these conditions the overall extrusion rate is 20 lbs/hour. A vacuum line is applied to two of three vent ports to extract water from the material during pelletization. Torque is 10%. The mixture is extruded into strands 0.3-0.8 cm in diameter and the strands are chopped to form pellets via a Conair pellitizer. The pellets are dried for 12 hours in a through air dryer at 150°F. The pellets are fed into a Hills 4-hole extruder test stand (Hills, Inc., West Melbourne, FL) with a Hills bicomponent sheath/core 4-hole spin pack. The equipment features two extruders that feed to a single spin head to produce bicomponent fibers. For bicomponent fibers, the thermoplastic composition as described above is fed into extruder 1. In the second extruders a polypropylene Profax™ PH835 (Basell) is used, under the following conditions:

| | Extruder Melt Pressure (psi) | Barrel Zone 1 (°C) | Barrel Zone 2 (°C) | Barrel Zone 3 (°C) | Extruder Pressure (psi) | Spin Head (°C) |
|---|---|---|---|---|---|---|
| Set Extruder 1 (°C) TPS | 1400 | 125 | 160 | 170 | 1500 | 175 |
| Set Extruder 2 (°C) PP | 1400 | 165 | 170 | 175 | 1500 | |

Fibers are collected through an attenuating air jet set at 20 psi. A total mass throughput of 0.75 g/hole-min is maintained. Adjusting the ratio of the melt pump speeds can produce sheath core fibers of different sheath thicknesses. The following sheath /core volume ratios are produced:

| Sheath (PP) (% volume) | Core (TPS) (% volume) |
|---|---|
| 5 | 95 |
| 10 | 90 |
| 15 | 85 |
| 20 | 80 |

The fibers are collected and dried overnight in a convection oven at 115°C . The fibers are subjected to the water stability test. All fibers pass the water stability test.

### Example 8

### Bicomponent fibers with PP

This example demonstrates blending and spinning of bicomponent fibers with water stability. The following materials are used to produce a thermoplastic composition:
6000 g Ethylex™ 2015 (Tate& Lyle, Decatur, IL)
1900 g Glycerol (Aldrich Chemicals, St. Louis, MO)
500g Polypropylene Profax™ PH835 (Basell, Elkton, MD)
500g Maleated Polypropylene G3003 (Eastman Chemicals, Kingsport, TN)
500 g Adipic acid (Solutia Chemicals, St. Louis, MO)
50 g Magnesium stearate (Aldrich Chemicals, St. Louis, MO)

The solid components are dry mixed in a Henschel Raw Material Mixer (Green Bay, WI) for 4 minutes at 1000 rpm. The mixture is then fed into a B&P Process System Twin Screw Extrusion Compounding System (Saginaw, MI) with 40 mm co-rotating screws. Glycerol is fed through a liquid feed port at a rate that maintains the desired composition (stated above). The screw speed is set at 90 rpm with the thermal profile as shown below:

| Temperature | zone 1 | zone 2 | zone 3 | zone 4 | zone 5 | zone 6 | zone 7 | zone 8 | zone 9 | die |
|---|---|---|---|---|---|---|---|---|---|---|
| Set(°C) | 85 | 85 | 100 | 145 | 155 | 160 | 160 | 160 | 140 | 100 |
| Actual (°C) | 83 | 83 | 85 | 138 | 138 | 144 | 155 | 147 | 133 | 98 |

At these conditions the overall extrusion rate is 20 lbs/hour. A vacuum line is applied to two of three vent ports to extract water from the material during pelletization. Torque is 10%. The mixture is extruded into strands 0.3-0.8 cm in diameter and the strands are chopped to form pellets via a Conair pellitizer. The pellets are dried for 12 hours in a through air dryer at 150°F. The pellets are fed into a Hills 4-hole extruder test stand (Hills, Inc., West Melbourne, FL) with a Hills bicomponent sheath/core 4-hole spin pack. The equipment features two extruders that feed to a single spin head to produce bicomponent fibers. For bicomponent fibers, the thermoplastic composition as described above is fed into extruder 1. In the second extruders a polypropylene Profax™ PH835 (Basell) is used, under the following conditions:

| | Extruder Melt Pressure (psi) | Barrel Zone 1 (°C) | Barrel Zone 2 (°C) | Barrel Zone 3 (°C) | Extruder Pressure (psi) | Spin Head (°C) |
|---|---|---|---|---|---|---|
| Set Extruder 1 (°C) TPS | 1400 | 125 | 160 | 170 | 1500 | 175 |
| Set Extruder 2 (°C) PP | 1400 | 165 | 170 | 175 | 1500 | |

Fibers are collected through an attenuating air jet set at 20 psi. A total mass throughput of 0.75 g/hole-min is maintained. Adjusting the ratio of the melt pump speeds can produce sheath core fibers of different sheath thicknesses. The following sheath /core volume ratios are produced:

| Sheath (PP) (% volume) | Core (TPS) (% volume) |
|---|---|
| 5 | 95 |
| 10 | 90 |
| 15 | 85 |
| 20 | 80 |

The fibers are collected and dried overnight in a convection oven at 115 °C. The fibers are subjected to the water stability test. All fibers pass the water stability test.

### Example 9

### Binder fibers

This example demonstrates additional blending and spinning of binder fibers with water stability. The following materials are used:
6000 g Ethylex™ 2015 (Tate & Lyle, Decatur, IL)
2500 g Glycerol (Aldrich Chemicals, St. Louis, MO)
500 g Maleated polypropylene (Eastman Chemicals, Kingsport, TN)
50 g Magnesium stearate (Aldrich Chemicals, St. Louis, MO)

The starch, adipic acid, maleated polypropylene and magnesium stearate are dry mixed in a Henschel Raw Material Mixer (Green Bay, WI) for 4 minutes at 1000 rpm. The mixture is then fed into a B&P Process System Twin Screw Extrusion Compounding System (Saginaw, MI) with 40 mm co-rotating screws. Glycerol is fed through a liquid feed port at a rate that maintains the desired composition (stated above). The screw speed is set at 90 rpm with the thermal profile as shown below:

| Temperature | zone 1 | zone 2 | zone 3 | zone 4 | zone 5 | zone 6 | zone 7 | zone 8 | zone 9 | die |
|---|---|---|---|---|---|---|---|---|---|---|
| Set(°C) | 85 | 85 | 100 | 145 | 155 | 160 | 160 | 160 | 140 | 100 |
| Actual (°C) | 83 | 83 | 85 | 138 | 138 | 144 | 155 | 147 | 133 | 98 |

At these conditions the overall extrusion rate is 20 lbs/hour. A vacuum line is applied to two of three vent ports to extract water from the material during pelletization. Torque is 10%. The mixture is extruded into strands 0.3-0.8 cm in diameter and the strands are chopped to form pellets via a Conair pellitizer. The pellets are dried for 12 hours in a through air dryer at 150°F. The pellets are fed into a Hills 4-hole extruder test stand (Hills, Inc., West Melbourne, FL) with a Hills bicomponent sheath/core 4-hole spin pack. The equipment features two extruders that feed to a single spin head to produce bicomponent fibers. For single component fibers, both extruders are set to identical conditions as follows and the same material is fed into both extruders:

| | Extruder Melt Pressure (psi) | Barrel Zone 1 (°C) | Barrel Zone 2 (°C) | Barrel Zone 3 (°C) | Extruder Pressure (psi) | Melt Pump Speed (rpm) | Spin Head (°C) |
|---|---|---|---|---|---|---|---|
| Set Extruder 1 (°C) | 1400 | 125 | 160 | 170 | 1500 | 464 | 165 |
| Set Extruder 2 (°C) | 1400 | 125 | 160 | 170 | 1500 | 464 | |

Fibers are collected in on a screen through an attenuating air jet at a mass throughput of 0.8 g/hole-min. The air jet is set at 20 psi. The Thermoplastic starch fibers are collected, chopped with a knife to lengths approximately 2 cm. The starch fibers are mixed with unbonded staple polyester fibers (Wellman, Fort Mill, SC) at a ratio of 10:1 by weight polyester to starch web for a total basis weight of approximately 50 gsm. The unbonded web is placed in a Carver™ Press and pressed at 1000 psi at 165°C for 10 minutes between Teflon sheets. The web is removed and allowed to cool. The web is dried overnight in a vacuum oven at 115°C . The web is subjected to the following water stability test: A 5cm x 5cm web is placed in 1000ml of water and allowed to soak for 24 hours. The web is removed and if it remains intact, it is said to pass the water stability test. The dried web passes the water stability test.

### Example 10

### Fibers using catalyst

This example demonstrates blending and spinning of bicomponent fibers with water stability. The following materials are used to produce a thermoplastic composition:
6000 g Ethylex™ 2015 (Tate& Lyle, Decatur, IL)
1900 g Glycerol (Aldrich Chemicals, St. Louis, MO)
500g Polypropylene Profax™ PH835 (Basell, Elkton, MD)
500g Maleated Polypropylene G3003 (Eastman Chemicals, Kingsport, TN)
1.9g p-Toluenesulfonic acid (Aldrich Chemicals, St. Louis, MO)
500 g Adipic acid (Solutia Chemicals, St. Louis, MO)
50 g Magnesium stearate (Aldrich Chemicals, St. Louis, MO)

The solid components are dry mixed in a Henschel Raw Material Mixer (Green Bay, WI) for 4 minutes at 1000 rpm. The mixture is then fed into a B&P Process System Twin Screw Extrusion Compounding System (Saginaw, MI) with 40 mm co-rotating screws. Glycerol is fed through a liquid feed port at a rate that maintains the desired composition (stated above). The screw speed is set at 90 rpm with the thermal profile as shown below:

| Temperature | zone 1 | zone 2 | zone 3 | zone 4 | zone 5 | zone 6 | zone 7 | zone 8 | zone 9 | die |
|---|---|---|---|---|---|---|---|---|---|---|
| Set (°C) | 85 | 85 | 100 | 145 | 155 | 160 | 160 | 160 | 140 | 100 |
| Actual (°C) | 83 | 83 | 85 | 138 | 138 | 144 | 155 | 147 | 133 | 98 |

At these conditions the overall extrusion rate is 20 lbs/hour. A vacuum line is applied to two of three vent ports to extract water from the material during pelletization. Torque is 10%. The mixture is extruded into strands 0.3-0.8 cm in diameter and the strands are chopped to form pellets via a Conair pellitizer. The pellets are dried for 12 hours in a through air dryer at 150°F. The pellets are fed into a Hills 4-hole extruder test stand (Hills, Inc., West Melbourne, FL) with a Hills bicomponent sheath/core 4-hole spin pack. The equipment features two extruders that feed to a single spin head to produce bicomponent fibers. For bicomponent fibers, the thermoplastic composition as described above is fed into extruder 1. In the second extruders a polypropylene Profax™ PH835 (Basell) is used, under the following conditions:

| | Extruder Melt Pressure (psi) | Barrel Zone 1 (°C) | Barrel Zone 2 (°C) | Barrel Zone 3 (°C) | Extruder Pressure (psi) | Spin Head (°C) |
|---|---|---|---|---|---|---|
| Set Extruder 1 (°C) TPS | 1400 | 125 | 160 | 170 | 1500 | 175 |
| Set Extruder 2 (°C) PP | 1400 | 165 | 170 | 175 | 1500 | |

Fibers are collected through an attenuating air jet set at 20 psi. A total mass throughput of 0.75 g/hole-min is maintained. Adjusting the ratio of the melt pump speeds can produce sheath core fibers of different sheath thicknesses. The following sheath /core volume ratios are produced:

| Sheath (PP) (% volume) | Core (TPS). (% volume) |
|---|---|
| 5 | 95 |
| 10 | 90 |
| 15 | 85 |
| 20 | 80 |

The fibers are collected and dried overnight in a convection oven at 115°C . The fibers are subjected to the water stability test. All fibers pass the water stability test.

### Example 11

This example demonstrates blending and spinning of bicomponent fibers with water stability comprising triglycerides. The following materials are used to produce a thermoplastic composition:
6000 g Ethylex™ 2015 (Tate& Lyle, Decatur, IL)
1900 g Glycerol (Aldrich Chemicals, St. Louis, MO)
500g Polypropylene Profax™ PH835 (Basell, Elkton, MD)
500g Maleated Polypropylene G3003 (Eastman Chemicals, Kingsport, TN)
500 g Adipic acid (Solutia Chemicals, St. Louis, MO)
500g Linseed oil (Aldrich Chemicals, St. Louis, MO)
50 g Magnesium stearate (Aldrich Chemicals, St. Louis, MO)

All the components except glycerol are mixed in a Henschel Raw Material Mixer (Green Bay, WI) for 4 minutes at 1000 rpm. The mixture is then fed into a B&P Process System Twin Screw Extrusion Compounding System (Saginaw, MI) with 40 mm co-rotating screws. Glycerol is fed through a liquid feed port at a rate that maintains the desired composition (stated above). The screw speed is set at 90 rpm with the thermal profile as shown below:

| Temperature | zone 1 | zone 2 | zone 3 | zone 4 | zone 5 | zone 6 | zone 7 | zone 8 | zone 9 | die |
|---|---|---|---|---|---|---|---|---|---|---|
| Set(°C) | 85 | 85 | 100 | 145 | 155 | 160 | 160 | 160 | 140 | 100 |
| Actual (°C) | 83 | 83 | 85 | 138 | 138 | 144 | 155 | 147 | 133 | 98 |

At these conditions the overall extrusion rate is 20 lbs/hour. A vacuum line is applied to two of three vent ports to extract water from the material during pelletization. Torque is 10%. The mixture is extruded into strands 0.3-0.8 cm in diameter and the strands are chopped to form pellets via a Conair pellitizer. The pellets are dried for 12 hours in a through air dryer at 150°F. The pellets are fed into a Hills 4-hole extruder test stand (Hills, Inc., West Melbourne, FL) with a Hills bicomponent sheath/core 4-hole spin pack. The equipment features two extruders that feed to a single spin head to produce bicomponent fibers. For bicomponent fibers, the thermoplastic composition as described above is fed into extruder 1. In the second extruders a polypropylene Profax™ PH835 (Basell) is used, under the following conditions:

| | Extruder Melt Pressure (psi) | Barrel Zone 1 (°C) | Barrel Zone 2 (°C) | Barrel Zone 3 (°C) | Extruder Pressure (psi) | Spin Head (°C) |
|---|---|---|---|---|---|---|
| Set Extruder 1 (°C) TPS | 1400 | 125 | 160 | 170 | 1500 | 175 |
| Set Extruder 2 (°C) PP | 1400 | 165 | 170 | 175 | 1500 | |

Fibers are collected through an attenuating air jet set at 20 psi. A total mass throughput of 0.75 g/hole-min is maintained. Adjusting the ratio of the melt pump speeds can produce sheath core fibers of different sheath thicknesses. The following sheath /core volume ratios are produced:

| Sheath (PP) (% volume) | Core (TPS) (% volume) |
|---|---|
| 5 | 95 |
| 10 | 90 |
| 15 | 85 |
| 20 | 80 |

The fibers are collected and dried overnight in a convection oven at 115°C . The fibers are subjected to the water stability test. All fibers pass the water stability test.

### Example 12

This example demonstrates blending and spinning of bicomponent fibers with water stability comprising triglycerides. The following materials are used to produce a thermoplastic composition:
6000 g Ethylex™ 2015 (Tate& Lyle, Decatur, IL)
1900 g Glycerol (Aldrich Chemicals, St. Louis, MO)
500g Polypropylene Profax™ PH835 (Basell, Elkton, MD)
500g Maleated Polypropylene G3003 (Eastman Chemicals, Kingsport, TN)
500 g Adipic acid (Solutia Chemicals, St. Louis, MO)
200g Soybean oil (Aldrich Chemicals, St. Louis, MO)
50 g Magnesium stearate (Aldrich Chemicals, St. Louis, MO)

All the components except glycerol are mixed in a Henschel Raw Material Mixer (Green Bay, WI) for 4 minutes at 1000 rpm. The mixture is then fed into a B&P Process System Twin Screw Extrusion Compounding System (Saginaw, MI) with 40 mm co-rotating screws. Glycerol is fed through a liquid feed port at a rate that maintains the desired composition (stated above). The screw speed is set at 90 rpm with the thermal profile as shown below:

| Temperature | zone 1 | zone 2 | zone 3 | zone 4 | zone 5 | zone 6 | zone 7 | zone 8 | zone 9 | die |
|---|---|---|---|---|---|---|---|---|---|---|
| Set(°C) | 85 | 85 | 100 | 145 | 155 | 160 | 160 | 160 | 140 | 100 |
| Actual (°C) | 83 | 83 | 85 | 138 | 138 | 144 | 155 | 147 | 133 | 98 |

At these conditions the overall extrusion rate is 20 lbs/hour. A vacuum line is applied to two of three vent ports to extract water from the material during pelletization. Torque is 10%. The mixture is extruded into strands 0.3-0.8 cm in diameter and the strands are chopped to form pellets via a Conair pellitizer. The pellets are dried for 12 hours in a through air dryer at 150°F. The pellets are fed into a Hills 4-hole extruder test stand (Hills, Inc., West Melbourne, FL) with a Hills bicomponent sheath/core 4-hole spin pack. The equipment features two extruders that feed to a single spin head to produce bicomponent fibers. For bicomponent fibers, the thermoplastic composition as described above is fed into extruder 1. In the second extruders a polypropylene Profax™ PH835 (Basell) is used, under the following conditions:

| | Extruder Melt Pressure (psi) | Barrel Zone 1 (°C) | Barrel Zone 2 (°C) | Barrel Zone 3 (°C) | Extruder Pressure (psi) | Spin Head (°C) |
|---|---|---|---|---|---|---|
| Set Extruder 1 (°C) TPS | 1400 | 125 | 160 | 170 | 1500 | 175 |
| Set Extruder 2 (°C) PP | 1400 | 165 | 170 | 175 | 1500 | |

Fibers are collected through an attenuating air jet set at 20 psi. A total mass throughput of 0.75 g/hole-min is maintained. Adjusting the ratio of the melt pump speeds can produce sheath core fibers of different sheath thicknesses. The following sheath /core volume ratios are produced:

| Sheath (PP) (% volume) | Core (TPS) (% volume) |
|---|---|
| 5 | 95 |
| 10 | 90 |
| 15 | 85 |
| 20 | 80 |

The fibers are collected and dried overnight in a convection oven at 115°C . The fibers are subjected to the water stability test. All fibers pass the water stability test

### Example 13

### Web from TPS fibers

TPS fiber prepared as in example 8 with a core sheath ratio of 95/5 TPS/PP. Webs of approximately 60 grams /m² are bonded via heated calender with diamond shaped pattern (1 mm in width , at 2mm intervals) at 60°C. The webs are dried in a oven at 115°C for 12 hours. A . 5cm x 5cm piece of the web is put into 1000ml of tap water and stirred at 30 rpm for 24 hours. The web is removed from the water dried in air for 24 hours then measured. The length and width dimension changes by no more than 15% and the web is essentially intact. The web is said to display water stability.

### Comparative Example 14

### Web from non-water stable TPS fibers

The following materials are used to produce a thermoplastic composition:
6000 g Ethylex™ 2015 (Tate& Lyle, Decatur, IL)
2500 g Glycerol (Aldrich Chemicals, St. Louis, MO)

The starch is fed into a B&P Process System Twin Screw Extrusion Compounding System (Saginaw, MI) with 40 mm co-rotating screws. Glycerol is fed through a liquid feed port at a rate that maintains the desired composition (stated above). The screw speed is set at 90 rpm with the thermal profile as shown below:

| Temperature | zone 1 | zone 2 | zone3 | zone 4 | zone 5 | zone 6 | zone 7 | zone 8 | zone 9 | die |
|---|---|---|---|---|---|---|---|---|---|---|
| Set(°C) | 85 | 85 | 100 | 145 | 155 | 160 | 160 | 160 | 140 | 100 |
| Actual (°C) | 83 | 83 | 85 | 138 | 138 | 144 | 155 | 147 | 133 | 98 |

At these conditions the overall extrusion rate is 20 lbs/hour. A vacuum line is applied to two of three vent ports to extract water from the material during pelletization. Torque is 10%. The mixture is extruded into strands 0.3-0.8 cm in diameter and the strands are chopped to form pellets via a Conair pellitizer. The pellets are dried for 12 hours in a through air dryer at 150°F. The pellets are fed into a Hills 4-hole extruder test stand (Hills, Inc., West Melbourne, FL) with a Hills bicomponent sheath/core 4-hole spin pack. The equipment features two extruders that feed to a single spin head to produce bicomponent fibers. For bicomponent fibers, the thermoplastic composition as described above is fed into extruder 1. In the second extruders a polypropylene Profax™ PH835 (Basell) is used, under the following conditions:

| | Extruder Melt Pressure (psi) | Barrel Zone 1 (°C) | Barrel Zone 2 (°C) | Barrel Zone 3 (°C) | Extruder Pressure (psi) | Spin Head (°C) |
|---|---|---|---|---|---|---|
| Set Extruder 1(°C) TPS | 1400 | 125 | 160 | 170 | 1500 | 175 |
| Set Extruder 2(°C) TPS | 1400 | 165 | 170 | 175 | 1500 | |

Fibers are collected through an attenuating air jet set as 20 psi. a total mass throughput of 0.75 g/hole-min is maintained. Adjusting the ratio of the melt pump speeds can produce sheath core fibers of different sheath thicknesses. The following sheath/core volume ratio is produced:

| Sheath (PP) (% volume) | Core (TPS) (% volume) |
|---|---|
| 5 | 95 |

Webs of approximately 60 grams/m² are bonded via heated calender with diamond shaped pattern (1 mm in width, at 2 mm interval) at 165°C. The webs are dried in a oven at 115°C for 12 hours. A 5cmx5cm piece of the web is put into 1000ml of tap water and stirred at 30 rpm for 24 hours. The web is removed from the water dried in air for 24 hours then measured. The length and width dimension changes by more than 15% and the web is not essentially intact with missing pieces. The web is said to not display water stability.

## Claims

1. A method of making water stable fibers comprising the steps of:
a. forming a mixture of:
i. destructured starch, preferably destructured in situ;
ii. polyhydric alcohol having alcohol functional groups; and
iii. acid having at least one functional group selected from the group consisting of: carboxylic acid; carboxylic anhydride; and combinations thereof;
b. extruding said mixture through a spinneret at elevated temperature, preferably above 90°C. to form fibers; and
c. inducing an ester condensation reaction between at least a portion of said polyhydric alcohol and said acid, wherein water is a product of said reaction and wherein said reaction is induced before or during the formation of the fiber
wherein as used herein the term water stable means the fibers remain intact after two weeks in 200 ml of tap water at room temperature according to the procedure disclosed in the description.

2. A method according to claim 1, wherein said acid is selected from the group consisting of: monoacid; diacid; polyacid; polymer comprising at least one acid moiety; co-polymer comprising at least one acid moiety; anhydrides thereof; and combinations thereof.

3. A method according to claim 1 or claim 2, wherein the mixture of (a) further comprises a triglyceride.

4. A method according to claim 3, wherein the step c. further comprises inducing a transesterification reaction between said polyhydric alcohol and said trigyceride.

5. A method according to any one of claims 1 to 4, wherein said polyhydric alcohol and said acid are partially provided to said mixture in the form of a pre-polymer.

6. A method according to any one of claims 1 to 5, wherein said polyhydric alcohol and said triglyceride are partially provided to said mixture in the form of a pre-polymer.

7. The method according to any one of claims 1 to 3, wherein said ester condensation reaction is induced before formation of the fibers but said reaction is not completed to such an extent that a gel of the reaction products is formed before formation of said fibers.

8. A method according to any one of claims 1 to 7, wherein the fibers after step (b) are heated at a temperature of from 100° to 180°C.

9. A fiber comprising a thermoplastic starch composition, said composition comprising:
a. at least 50wt.%, by weight of the composition, of destructured starch, preferably destructured in situ; and
b. ester condensation products formed from a chemical reaction in a reactant mixture, said reactant mixture being compounded with said destructured starch and said reactant mixture comprising:
i. polyhydric alcohol having alcohol functional groups, preferably selected from the group consisting of: glycerol; glycol; sugar; sugar alcohol; and combinations thereof; more preferably glycerol; and
ii. acid with at least one functional group selected from the group consisting of: carboxylic acid; carboxylic acid anhydride; and combinations thereof;
said functional groups being present in said reactant mixture in a molar ratio of said alcohol functional groups to said at least one acid functional group of from 1:1 to 200;1
wherein said fiber is water stable, which as used herein means the fiber remains intact after two weeks in 200ml of tap water at room temperature according to the procedure disclosed in the description.

10. A fiber according to claim 9, wherein said compositions further comprise additional polymer selected from the group consisting of: polyhydroxyalkanoate; polyvinyl alcohol; polyethylene; polypropylene; maleated polyethylene, maleated polypropylene; polyethylene terephthalate; polylactic acid; modified polypropylene; nylon; caprolactone; and combinations thereof.

11. A fiber according to claim 9 or claim 10, wherein said fiber is biodegradable, and said compositions preferably further comprise an additional polymer selected from the group consisting of: polyvinyl alcohol; ester poly condensates, aliphatic/aromatic polyesters; and combinations thereof.

12. A fiber according to any of claims 9 to 11, wherein said fiber is selected from the group consisting of monocomponent fibers; multicomponent fibers; multiconstituent fibers; and, combinations thereof.

13. A nonwoven fabric comprising fiber according to any of claims 9 to 12.

14. A personal hygiene article comprising fiber according to any one of claims 9 to 12.

15. An absorbent article comprising fiber according to any one of claims 9 to 12.

## Patentansprüche

1. Verfahren zum Herstellen von wasserbeständigen Fasern, umfassend die folgenden Schritte:
A. Bilden einer Mischung aus:
I. entstrukturierter, vorzugsweise in situ entstrukturierter Stärke;
II. Polyalkohol mit funktionellen Alkoholgruppen; und
III. Säure mit mindestens einer funktionellen Gruppe, die ausgewählt ist aus der Gruppe, bestehend aus:
Carbonsäure; Carbonsäureanhydrid; und Kombinationen davon;
B. Extrudieren der Mischung durch eine Spinndüse bei erhöhter Temperatur, vorzugsweise über 90 °C, zum Bilden von Fasern; und
C. Auslösen einer Esterkondensationsreaktion zwischen mindestens einem Teil des Polyalkohols und der Säure, wobei Wasser ein Produkt der Reaktion ist und wobei die Reaktion vor oder während der Bildung der Faser ausgelöst wird
wobei der Ausdruck wasserbeständig, wie hier verwendet, bedeutet, dass die Fasern nach zwei Wochen in 200 ml Leitungswasser bei Raumtemperatur gemäß dem in der Beschreibung offenbarten Verfahren intakt bleiben.

2. Verfahren nach Anspruch 1, wobei die Säure ausgewählt ist aus der Gruppe bestehend aus Monosäure; Disäure; Polysäure; Polymer, das mindestens eine Säureeinheit umfasst; Copolymer, das mindestens eine Säureeinheit umfasst; Anhydriden davon; und Kombinationen davon.

3. Verfahren nach Anspruch 1 oder Anspruch 2, wobei die Mischung aus (a) ferner ein Triglycerid umfasst.

4. Verfahren nach Anspruch 3, wobei Schritt c. ferner das Auslösen einer Umesterungsreaktion zwischen dem Polyalkohol und dem Triglycerid umfasst.

5. Verfahren nach einem der Ansprüche 1 bis 4, wobei der Polyalkohol und die Säure der Mischung teilweise in Form eines Präpolymers bereitgestellt werden.

6. Verfahren nach einem der Ansprüche 1 bis 5, wobei der Polyalkohol und das Triglycerid der Mischung teilweise in Form eines Präpolymers bereitgestellt werden.

7. Verfahren nach einem der Ansprüche 1 bis 3, wobei die Esterkondensationsreaktion vor der Bildung der Fasern ausgelöst wird, jedoch die Reaktion nicht in einem derartigen Maße abgeschlossen wird, dass ein Gel der Reaktionsprodukte vor der Bildung der Fasern gebildet wird,

8. Verfahren nach einem der Ansprüche 1 bis 7, wobei die Fasern nach Schritt (b) auf eine Temperatur von 100 °C bis 180 °C erwärmt werden.

9. Faser, umfassend eine thermoplastische Stärkezusammensetzung, wobei die Zusammensetzung Folgendes umfasst:
A. mindestens 50 Gew.-% der Zusammensetzung entstrukturierte Stärke, vorzugsweise in situ entstrukturierte Stärke; und
B. Esterkondensationsprodukte, die aus einer chemischen Reaktion in einer Reagenzmischung gebildet werden, wobei die Reagenzmischung mit der entstrukturierten Stärke compoundiert wird und die Reagenzmischung Folgendes umfasst:
I. Polyalkohol mit funktionellen Alkoholgruppen, vorzugsweise ausgewählt aus der Gruppe bestehend aus: Glycerol; Glycol; Zucker; Zuckeralkohol; und Kombinationen davon; mehr bevorzugt Glycerol; und
II. Säure mit mindestens einer funktionellen Gruppe, die ausgewählt ist aus der Gruppe bestehend aus: Carbonsäure; Carbonsäureanhydrid; und Kombinationen davon;
wobei die funktionellen Gruppen in der Reagenzmischung in einem Molverhältnis der funktionellen Alkoholgruppen zu der mindestens einen funktionellen Säuregruppe von 1:1 1 bis 200:1 vorhanden sind
wobei die Faser wasserbeständig ist, was, wie hier verwendet, bedeutet, dass die Faser nach zwei Wochen in 200 ml Leitungswasser bei Raumtemperatur gemäß dem in der Beschreibung offenbarten Verfahren intakt bleibt.

10. Faser nach Anspruch 9, wobei die Zusammensetzungen ferner ein zusätzliches Polymer umfassen, das ausgewählt ist aus der Gruppe bestehend aus: Polyhydroxyalkanoat; Polyvinylalkohol; Polyethylen; Polypropylen; mit Maleinsäure behandeltem Polyethylen; mit Maleinsäure behandeltem Polypropylen; Polyethylenterephthalat; Polymilchsäure; modifiziertem Polypropylen; Nylon; Caprolacton; und Kombinationen davon.

11. Faser nach Anspruch 9 oder Anspruch 10, wobei die Faser biologisch abbaubar ist und die Zusammensetzungen ferner vorzugsweise ein zusätzliches Polymer umfassen, das ausgewählt ist aus der Gruppe bestehend aus: Polyvinylalkohol; Esterpolykondensaten; aliphatischen/aromatischen Polyestern; und Kombinationen davon.

12. Faser nach einem der Ansprüche 9 bis 11, wobei die Faser ausgewählt ist aus der Gruppe bestehend aus Monokomponentenfasern; Multikomponentenfasern; mehrteiligen Fasern; und Kombinationen davon.

13. Vliesstoff, umfassend eine Faser nach einem der Ansprüche 9 bis 12.

14. Körperpflegeartikel, umfassend eine Faser nach einem der Ansprüche 9 bis 12.

15. Absorptionsartikel, umfassend eine Faser nach einem der Ansprüche 9 bis 12.

## Revendications

1. Procédé de fabrication de fibres stables dans l'eau comprenant les étapes consistant à :
a. former un mélange de :
I. un amidon déstructuré, de préférence déstructuré in situ ;
II. un alcool polyhydrique ayant des groupes fonctionnels alcool ; et
III. un acide ayant au moins un groupe fonctionnel choisi dans le groupe constitué de :
un acide carboxylique ; un anhydride carboxylique ; et leurs combinaisons ;
b. extruder ledit mélange à travers une buse à filer à température élevée, de préférence supérieure à 90 °C, de façon à former des fibres ; et
c. amorcer une réaction de condensation d'ester entre au moins une partie dudit alcool polyhydrique et dudit acide, dans lequel l'eau est un produit de ladite réaction et dans lequel ladite réaction est amorcée avant ou pendant la formation de la fibre
dans lequel, tel qu'il est utilisé ici, le terme stable dans l'eau signifie que les fibres demeurent intactes après deux semaines dans 200 mL d'eau du robinet à la température ambiante selon la procédure décrite dans la description.

2. Procédé selon la revendication 1, dans lequel ledit acide est choisi dans le groupe constitué d'un monoacide ; un diacide ; un polyacide ; un polymère comprenant au moins un fragment acide ; un copolymère comprenant au moins un fragment acide ; leurs anhydrides ; et leurs combinaisons.

3. Procédé selon la revendication 1 ou la revendication 2, dans lequel le mélange de (a) comprend, en outre, un triglycéride.

4. Procédé selon la revendication 3, dans lequel l'étape c. comprend, en outre, l'amorçage d'une réaction de transestérification entre ledit alcool polyhydrique et ledit triglycéride.

5. Procédé selon l'une quelconque des revendications 1 à 4, dans lequel ledit alcool polyhydrique et ledit acide sont partiellement fournis audit mélange sous la forme d'un prépolymère.

6. Procédé selon l'une quelconque des revendications 1 à 5, dans lequel ledit alcool polyhydrique et ledit triglycéride sont partiellement fournis audit mélange sous la forme d'un prépolymère.

7. Procédé selon l'une quelconque des revendications 1 à 3, dans lequel ladite réaction de condensation d'ester est amorcée avant la formation des fibres, mais ladite réaction n'est pas achevée à un point tel qu'un gel des produits de réaction est formé avant la formation desdites fibres,

8. Procédé selon l'une quelconque des revendications 1 à 7, dans lequel les fibres après l'étape (b) sont chauffées à une température allant de 100 °C à 180 °C.

9. Fibre comprenant une composition d'amidon thermoplastique, ladite composition comprenant :
a. au moins 50 % en poids de la composition, d'amidon déstructuré, de préférence déstructuré in situ ; et
b. des produits de condensation d'ester formés à partir d'une réaction chimique dans un mélange de réactifs, ledit mélange de réactifs étant mélangé avec ledit amidon déstructuré et ledit mélange de réactifs comprenant :
i. un alcool polyhydrique ayant des groupes fonctionnels alcool, choisi de préférence dans le groupe constitué de : glycérol ; glycol ; sucre ; alcool glucidique ; et leurs combinaisons ; plus préférablement le glycérol ; et
ii. un acide avec au moins un groupe fonctionnel choisi dans le groupe constitué de : acide carboxylique ; anhydride d'acide carboxylique ; et leurs combinaisons ;
lesdits groupes fonctionnels étant présents dans ledit mélange de réactifs dans un rapport molaire desdits groupes fonctionnels alcool sur ledit au moins un groupe fonctionnel acide allant de 1:1 à 200:1
où ladite fibre est stable dans l'eau, ce qui, tel qu'il est utilisé ici, signifie que la fibre demeure intacte après deux semaines dans 200 mL d'eau du robinet à la température ambiante selon la procédure décrite dans la description.

10. Fibre selon la revendication 9, dans laquelle lesdites compositions comprennent en outre un polymère supplémentaire choisi dans le groupe constitué de : un polyhydroxyalcanoate ; un alcool polyvinylique ; un polyéthylène ; un polypropylène ; un polyéthylène maléaté ; un polypropylène maléaté ; un polyéthylène téréphtalate ; un acide polylactique ; un polypropylène modifié ; un nylon ; un caprolactone ; et leurs combinaisons.

11. Fibre selon la revendication 9 ou la revendication 10, où ladite fibre est biodégradable, et lesdites compositions comprennent de préférence, en outre, un polymère supplémentaire choisi dans le groupe constitué de : un alcool polyvinylique ; des poly-condensats d'ester ; des polyesters aliphatiques/aromatiques ; et leurs combinaisons.

12. Fibre selon l'une quelconque des revendications 9 à 11, où ladite fibre est choisie dans le groupe constitué de fibres à monocomposant ; fibres à multicomposants ; fibre à multi-constituants ; et leurs combinaisons.

13. Étoffe non tissée comprenant une fibre selon l'une quelconque des revendications 9 à 12.

14. Article d'hygiène personnelle comprenant une fibre selon l'une quelconque des revendications 9 à 12.

15. Article absorbant comprenant une fibre selon l'une quelconque des revendications 9 à 12.
